# EUROPEAN PATENT APPLICATION

(11) **EP 3 108 806 A1**
(43) Date of publication of application: **28.12.2016**
(21) Application number: 16175418.9
(22) Date of filing: 21.06.2016
(51) Int. Cl.: A61B 5/00, A61B 5/145, A61B 5/01, A61B 5/1172, A61B 10/00, A61N 1/04

(54) **DETECTION APPARATUS**

(30) Priority: 22.06.2015 GB 201510909
(71) Applicant: Surescreen Diagnostics Limited, Derby, Derbyshire DE1 3QB (GB)
(72) Inventor: Campbell, James Gordon, Derby, DE1 3QB (GB)
(74) Representative: Sales, Robert Reginald

(57) **Abstract**

A detection apparatus 10 for detecting the presence and amount of one of a number of materials within a person's body includes a flexible detection sleeve 28 which fits over a person's finger 32, and analysis means which may include a spectrometer head 40 for detecting for the various specified substances in transdermal exudates and/or interstitial fluid of the epidermis.

## Description

This invention concerns a detection apparatus, and a method of detection, and particularly but not exclusively apparatus and a method for detecting specified substances in transdermal exudates and/or interstitial fluid of the epidermis.

A number of medical diagnostic systems are already in existence. It is generally desirable for these to be as non invasive as possible and to require a minimum amount of medical intervention. Such diagnostic systems may relate to the use of alcohol or other drugs, or to the detection of particular diseases or conditions. Early detection of conditions and diseases is preferable and can lead to better treatment.

Conventionally alcohol has been tested using breathalysers to detect ethanol in breath. Breathalysers are however not useable with all persons. For instance, it may not be possible to use breathalysers with asthmatics, those with limited lung function and those who are incapacitated or who are unconscious, perhaps after an accident or other situation. Also the use of breathalysers is often associated with police activity and may not be considered appropriate to a work environment, where it may be required to discreetly check whether a person is fit for work.

According to a first aspect of the invention there is provided detection apparatus, the apparatus including a flexible detection sleeve locatable over a person's finger, and analysis means for detecting the presence of a specified substance or substances in the transdermal exudates and/or interstitial fluid of the epidermis from the person's finger.

The detection apparatus may be configured such that the detection sleeve towards a proximal open end thereof, substantially provides a seal around a person's finger located therein. The detection apparatus may be configured such that a chamber area is provided at the distal end of the detection sleeve to receive transdermal exudates from the person's finger.

The analysis means may be configured such that analysis of transdermal exudates and interstitial fluid of the epidermis may be carried out together or separately.

The detection sleeve may be made of a plastics material and may be disposable after each use.

The detection sleeve may taper inwardly away from the proximal open end.

Inward projections may be provided in the detection sleeve to apply pressure to a person's finger therein. The inward projections may be in the form of a plurality of discrete projections, which may be rounded. Alternatively, or in addition, inner ribs may be provided in the detection sleeve. The projections may extend into the detection sleeve by between 1 and 6mm, and more particularly between 2 and 4mm. The inward projections may be provided at least in the part of the detection sleeve corresponding to the finger print area of a person's finger therein.

The detection sleeve may be made of an electrically conducting material, and the apparatus may include an arrangement for applying an electrical current to the detection sleeve. The current may be iontophoretic, and may have a voltage of between 5 and 50mV and more particularly between 10 and 25mV, and may be substantially 15mV.

The apparatus may be arranged such that the polarity of the electrical current can be reversed. The apparatus may include an electrically conducting portion which a person can touch with another part of their hand to complete an electrical circuit.

The apparatus may be configured such that the chamber area has a volume of between 0.1 and 10ml, more desirably between 0.5 and 5ml, and more particularly between 0.5 and 1.5ml.

The detection sleeve may contain a substance which will promote the production of transdermal exudate. This substance may consist of a formulation including one or more of the following; niacin, tetrahydropiperine, methylsulphonylmethane (MSM), dexamethasone sodium phosphate, pilocarpine and the like.

The detection apparatus may include a body, and the body may be configured such that the detection sleeve can be located against, or extending into the body to enable spectroscopic analysis of the transdermal exudates and/or interstitial fluid.

A sensor may be provided to detect when a person's finger is placed is a required position in the detection sleeve for analysis to be carried out. The sensor may be a proximity or light sensor.

Urging means may be provided to urge a person's finger into a required position in the detection sleeve for analysis to be carried out.

The detection apparatus may include a cover member or an enclosed area which can surround the detection sleeve, and inflation means to provide increased pressure around the detection sleeve to urge same onto a person's finger. The cover member may be made of flexible plastics materials.

The analysis means may be configured to provide analysis by any of raman, UV, fluorescence, infrared detection, or visible light spectroscopic methods.

The apparatus may include a temperature detector. The temperature detector may detect the temperature of a person's finger in the detection sleeve and/or the temperature of transdermal exudates in the chamber area. The temperature detector may also detect the ambient or external atmospheric temperature conditions.

The apparatus may be configured such that if a temperature below a predetermined level is detected, an electric current is automatically applied to the sleeve, but if a temperature above the predetermined level is detected no electric current is applied to the sleeve.

The predetermined temperature may be between 30 and 37°C, and may be 32°C.

The temperature detector may include a thermal couple detector or an infra red detector.

The apparatus may include means for drawing vapour from the chamber area into the analysis means. The vapour drawing means may include a piston arrangement and/or a bellows arrangement.

The apparatus may be configured such that the detection sleeve connects directly to the analysis means. Alternatively, a passage may extend from the sleeve to the analysis means.

The analysis means may be arranged to detect any of the following: ethanol, acetaldehyde, trimethylamine, trans-3-methyl-2 hexanoic acid, pentyl pentanoate, putrescene, glucose, drugs and medicines and their metabolites, such as oxazepine, benzoylecgonine, cotinine, tetrahydrocannibinol; hydration levels, HbA1c, cardiac markers, ions, or other biometric indicators such as neurochemicals including serotonin, dopamine and noradrenaline, oxytocin, cortisol.

For detecting ethanol, the analysis means may include a platinised fuel cell and/or infrared.

The analysis means may provide a quantitative analysis of one or more substances to be detected.

The body may include a display means.

The apparatus may also include fingerprint recognition means, and this may be provided on the detection sleeve or part of the body.

The apparatus may include timing means, and the apparatus may be configured such that the analysis means only operates after a set time interval after a person's finger has been inserted into the sleeve, so as to permit equilibration.

The apparatus may be configured such that two sequential analyses can be made and the results compared such that if the analyses differ beyond an inputtable permissible level, a failure signal is emitted.

According to a second aspect of the invention there is provided a method of detection of a substance, the method comprising using an apparatus according to any of the preceding paragraphs.

The method may comprise detecting for the presence of any of: ethanol, acetaldehyde, trimethylamine, trans-3-methyl-2 hexenoic acid, pentyl pentanoate, putrescene, glucose, drugs and medicines and their metabolites, oxazepine, benzoylecgonine, cotinine, tetrahydrocannibinol; hydration levels, HbA1c, cardiac markers, ions, or other biometric indicators such as neurochemicals including serotonin, dopamine and noradrenaline, oxytocin, cortisol.

The method may include providing a qualitative or quantitative detection of one or more substances.

The analysis may not take place until a set dwell period after a person's finger has been placed in the sleeve. The dwell period may be at least 60 seconds.

If more than one sequential analysis is carried out, a shorter dwell period may be applied to subsequent analyses.

Two sequential analyses may be made and the results compared subject to a predetermined permissible variation, with a signal provided when the variation has been exceeded and therefore the analysis has failed.

A person's fingerprint may be detected using the apparatus during analysis.

An electrical charge may be applied to the detection sleeve. The electrical charge may have a voltage of between 5 and 50mV and more particularly between 10 and 25mV and may be substantially 15mV.

The polarity of the electric charge may be varied.

The temperature of a person's finger and/or the transdermal exudate in the detection sleeve may be measured, and an electric charge automatically applied if the temperature is below a predetermined level, which predetermined level may be between 30 and 37°C, and may be 32°C.

The detection sleeve may be disposed off after each use.

A software calculation may be used to distinguish blood concentration based on the result produced by reading either the interstitial fluid of the epidermis, the transdermal exudate, temperature or other results produced by the device or a combination of these together.

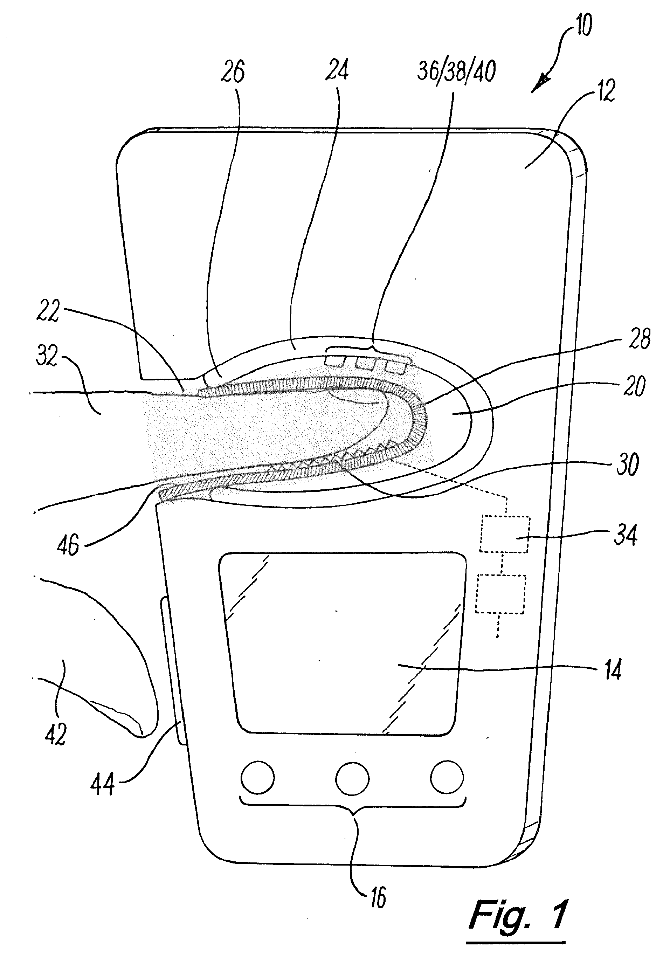

Embodiments of the present invention will now be described by way of example only and with reference to the accompanying drawings in which:-
Fig. 1 is a diagrammatic view of a detection apparatus according to the invention in use; and

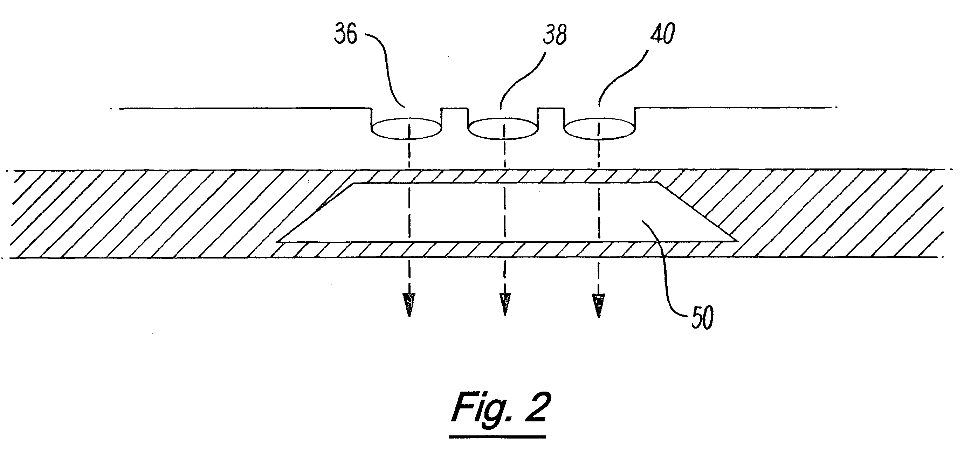

Fig. 2 is a more detailed view of part of the apparatus of Fig. 1.

The drawings show a detection apparatus 10 according to the invention. The apparatus 10 comprises a body 12 locating components for operating the apparatus 10. Provided on the exterior of the body 12 is a display screen 14 for indicating results and other features, and three control buttons 16. A metal panel 18 is also provided on a side of the body 12.

A chamber 20 is defined towards an upper part of the body 12, and has an opening 22. A lining 24 of a resilient material is provided around the chamber 20, and enlarged portions 26 of the resilient material are provided on opposite sides of the opening 22. A detection sleeve 28 is provided in the form of a disposable product which would be disposed off after each use and replaced by a fresh sterile sample.

The detection sleeve 28 is made of an electrically conductive flexible plastics material and is of a size to fit on a person's finger and to provide a substantial seal at the proximal end of the detection sleeve 28.

The detection sleeve 28 may taper towards the distal end thereof, and a plurality of projections 30 may be provided (only some of which are shown) on the inner surface of the detection sleeve 28 to apply pressure onto a person's finger 32 located in the detection sleeve 28. The projections 30 are in the form of rounded protruding spikes protruding around 2mm into the sleeve, and particularly over the area thereof corresponding to a person's fingerprint area. The projections are provided at a spacing of around 3mm.

An air supply 34 is provided into the chamber 20 spaced from the opening 22. In use the person's finger 32 will be placed in the detection sleeve 28, and located in the chamber 20. The sleeve 28 may be first located on a finger 32 before location in the chamber 20. A substantial seal will be produced around the finger at the proximal end, by the enlarged portions 26.

Sensors 36 and 38 for measuring respectively temperature and also the proximity of a person's finger 32 are provided on the inside of the chamber 20. A spectrometer head 40 is also provided on the inside of the chamber 20 which may operate by raman, uv, flourescence, infra red or visible light spectroscopy, to analyse transdermal exudates and/or interstitial fluid of the epidermis from a finger 32.

A window 50 is provided in the sleeve 28 to allow the sensors 36, 38 and spectroscopy head 40 to read directly onto the skin of the finger 32. The window may be provided by a material which does not interfere with spectroscopy measurements. The proximity sensor 38 detects whether a finger 32 is correctly located in the chamber 20.

Transdermal exudates, i.e. sweat, and/or institial fluid of the epidermis are emitted from the finger 32 carrying potentially volatile materials to be detected, and gather in the closed distal end of the detection sleeve 28. The detection sleeve 28 may be configured such that a chamber area is provided at the distal end, and this will typically have a volume of around 0.1 ml.

The projections 30 on the detection sleeve 28 exert a modest pressure on the finger 32 squeezing the sweat pores, and particularly in the fingerprint area.

The temperature sensor 36 detects the temperature of vapour drawn from the finger 32, and the temperature sensing means may include an infra red sensor or a thermal couple. The temperature is measured for use in computation of quantitative results.

A small iontophoretic electric current may be applied to the electrically conductive detection sleeve 28 to promote exudation. Such a current may invariably be applied, or may only be applied if a temperature below a set level such as for instance 32°C is detected. Whilst the electric current promotes exudation, it cannot be felt by the person. Placing a part of the person's hand such as the thumb 42 against a metal panel 44 on the exterior of the body 12, completes the electrical circuit.

Once the finger 32 has been inserted into the chamber 20, a set time is counted down by the apparatus 10 to develop an equilibrium amount of transdermal exudate in the detection sleeve 28, and typically such time, and for example when detecting the presence of alcohol, may be 60 seconds. At the end of this time period the electrical current if applied is switched off and vapour witihin the detection sleeve 28 may be withdrawn down a pipe which may be the same as for the air supply 34, by a solenoid operated piston or bellows unit for analysis within the body 12.

If the apparatus 10 is being used to detect alcohol, ethanol is detected using a platinised fuel cell which oxidises the ethanol and converts it to water and carbon dioxide, producing a small electrical charge which is detected by the instrument. A quantitative result is given and displayed on the screen 14 and saved for subsequent use. The apparatus 10 includes a memory store feature, wired and/or wireless connectivity to allow data to be reported efficiently. Device 10 may also include a touch screen for operation.

The apparatus 10 may also detect alcohol through use of the spectroscopic sensor 40, reading the interstitial fluid of the epidermis.

A software calculation may be used to distinguish blood concentration based on the result produced by reading either the interstitial fluid of the epidermis, the transdermal exudate or a combination of the two.

This software calculation may use a formula based on the reading of two or more parameters (such as ethanol, acetaldehyde, trimethylamine, trans-3-methyl-2 hexenoic acid, pentyl pentanoate, putrescene, glucose, drugs and medicines and their metabolites, hydration levels, HbA1c, cardiac markers, ions and other parameters found in the transdermal exudate or interstitial fluid of the epidermis and time,) to work out the blood concentration. The device may also take two or more readings of one or multiple parameters over a set period of time to allow for further software calculations.

Formulas used may be based on a time lag variable between substances being in the blood, and reaching transdermal exudates and interstitial fluid of the epidermis. Formulas may also be used based on back calculating concentrations based on the live results that are generated from the device. The device may also take into account variables such as the donors age, weight, BMI, heart rate, hydration level, or any other relevant biometric information.

It is often required to carry out a second detection to validate the first detection. If so then the cycle is repeated, though generally a shorter dwell time than 60 seconds would be needed. Typically the result of the second detection can be obtained within 5 to 30 seconds and perhaps 15 seconds. The two readings will then be compared relative to an input allowable difference. If the difference is not exceeded then the result will be displayed. If however the difference has been exceeded an invalid result will be displayed.

Once the cycle has been completed the apparatus will proceed through a purge and cleaning procedure to make it ready for a next measurement. In general after use each detection sleeve 28 will be disposed of, and a new one used. Alternatively multiple use sleeve with appropriate cleaning regimes may be used.

If required the air supply 34 can supply air into the chamber 20 outside of the detection sleeve 28 to urge the detection sleeve 28 against the person's finger to secrete transfer more exudate. A fingerprint recognition panel 46 is provided at the opening 22 to assist with confirming the identity of a user.

Whilst the above description relates to detecting the presence of alcohol, a range of potential conditions can be detected by this invention as described above. For instance, the use of illegal drugs may be detected.

Potential medical conditions may be detected as follows. To detect ketonuresis, analysis for the volatile substance acetaldehyde may be carried out. To detect liver disease or trimethylamiuria, analysis may be made of trimethylamine. To detect schizophrenia, analysis may be made for trans-3-methyl-2 hexenoic acid. To detect diabetes, analysis may be made of pentyl pentanoate. To detect gangrene, analysis may be made for putrescene.

To detect glucose, it will be necessary to reverse the polarity of the electric current applied to the detection sleeve 22, and use a glucose sensitivity detector incorporated into the detection sleeve 22. Reversing the current encourages non-polar substances to migrate through the finger for detection.

In using this apparatus to detect alcohol, useful and different results may be achieved relative to measuring breath alcohol. Whilst the quantity of ethanol excreted by skin is around 1 % of that found in breath alcohol testing, the emission from skin is delayed by some five hours compared with blood levels.

Results may be reported either qualitatively or quantitatively for all applications.

Apparatus according to the invention can be advantageous in avoiding eroneous measurements. For instance in the case of ethanol, as ethanol is very volatile, unless a person's finger had been contaminated with ethanol very shortly before a test, the ethanol will have evaporated for example from a hand antiseptic wash or otherwise, and will therefore not feature in the test. This may mean that the finger of a typical user does not require washing, wiping or any special preparation prior to the test.

The inclusion of two sequential tests and their comparison helps to ensure that that vapour equilibrium has occurred and thus the test is valid. Also such a validation helps to remove the possibility of a person cheating a test, as it is extremely unlikely that they could produce the same erroneous results twice.

In some instances, a single test will be sufficient to provide a rapid screening for instance alcohol, and this could for instance be applied prior to a driver to see whether they are fit or allowed to drive, and could be connected to an automatic system for preventing starting of a vehicle. If a more accurate result is required, then sequential results can be applied with only small allowed variants.

A wide range of modifications may be made without departing from the scope of the invention. For instance, different protrusions may be provided in the detection sleeve, and these could for instance be in the form of ribbing. As indicated the invention may be used to detect a wide range of materials.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. Detection apparatus, the apparatus (10) including a flexible detection sleeve (28) locatable over a person's finger (32), and analysis means for detecting the presence of a specified substance or substances in the transdermal exudates and/or interstitial fluid of the epidermis from the person's finger (32).

2. Detection apparatus according to claim 1, **characterised in that** the detection sleeve is made of an electrically conducting material, and the apparatus includes an arrangement for applying an electrical current to the derection sleeve.

3. Detection apparatus according to claims 1 or 2, **characterised in that** the detection sleeve contains a substance which will promote the production of transdermal exudate.

4. Detection apparatus according to any of the preceding claims, **characterised in that** the detection apparatus includes a body, and the body is configured such that the detection sleeve can be located against, or extending into the body to enable spectroscopic analysis of the transdermal exudates and/or interstitial fluid.

5. Detection apparatus according to claim 4, **characterised in that** the analysis means is configured to provide analysis by any of raman, UV flourescence, infrared detection, or visible light spectroscopic methods.

6. Detection apparatus according to any of the preceding claims, **characterised in that** urging means is provided to urge a person's finger into a required position in the detection sleeve for analysis to be carried out.

7. Detection apparatus according to any of the preceding claims, **characterised in that** the apparatus includes a temperature detector.

8. Detection apparatus according to claim 7 when dependent on claim 2, **characterised in that** the apparatus is configured such that if a temperature below a predetermined level is detected, an electric current is automatically applied to the sleeve, but if a temperature above the predetermined level is detected no electric current is applied to the sleeve.

9. Detection apparatus according to any of the preceding claims, **characterised in that** the analysis means is arranged to detect any of the following: ethanol, acetaldehyde, trimethylamine, trans-3-methyl-2 hexanoic acid, pentyl pentanoate, putrescene, glucose, drugs and medicines and their metabolites, such as oxazepine, benzoylecgonine, cotinine, tetrahydrocannibinol; hydration levels, HbA1c, cardiac markers, ions, or other biometric indicators such as neurochemicals including serotonin, dopamine and noradrenaline, oxytocin, cortisol.

10. Detection apparatus according to any of the preceding claims, **characterised in that** the apparatus also includes fingerprint recognition means.

11. Detection apparatus according to any of the preceding claims, **characterised in that** the apparatus includes timing means, and the apparatus is configured such that the analysis means only operates after a set time interval after a person's finger has been inserted into the sleeve, so as to permit equilibration.

12. A method of detection of a substance, the method comprising using an apparatus according to any of the preceding paragraphs.

13. A method according to claim 12, **characterised in that** the method comprises detecting for the presence of any of: ethanol, acetaldehyde, trimethylamine, trans-3-methyl-2 hexenoic acid, pentyl pentanoate, putrescene, glucose, drugs and medicines and their metabolites, oxazepine, benzoylecgonine, cotinine, tetrahydrocannibinol; hydration levels, HbA1c, cardiac markers, ions, or other biometric indicators such as neurochemicals including serotonin, dopamine and noradrenaline, oxytocin, cortisol.

14. A method according to claims 12 or 13, characterisd in that two sequential analyses are made and the results compared subject to a predetermined permissible variation, with a signal provided when the variation has been exceeded and therefore the analysis has failed.

15. A method according to any of claims 12 to 14, **characterised in that** a software calculation is used to distinguish blood concentration based on the result produced by reading any of the interstitial fluid of the epidermis, the transdermal exudate, temperature or other results produced by the device or a combination of these together.
